# EUROPÄISCHE PATENTSCHRIFT

(11) **EP 2 299 157 B1**
(45) Veröffentlichungstag und Bekanntmachung des Hinweises auf die Patenterteilung: **08.02.2012**
(21) Anmeldenummer: 10005095.4
(22) Anmeldetag: 14.05.2010
(51) Int. Cl.: F16L 37/084, F16L 37/40

(54) **Verschließbare Vorrichtung und Spülverfahren**
Closable device and rinsing method
Dispositif verrouillable et procédé de rinçage

(30) Priorität: 18.09.2009 DE 102009042231
(43) Veröffentlichungstag der Anmeldung: 23.03.2011
(73) Patentinhaber: Simmoteit, Robert, 72414 Rangendingen (DE)
(72) Erfinder: Simmoteit, Robert, 72414 Rangendingen (DE)

(56) Entgegenhaltungen:
- EP-A1- 0 813 018
- EP-A1- 2 088 358
- GB-A- 1 343 903
- GB-A- 1 466 242
- US-A- 2 457 052

## Beschreibung

### Gebiet der Erfindung

Die Erfindung betrifft eine verschließbare Vorrichtung zum Spülen, Anschließen und Koppeln, die insbesondere im Bereich der medizinischen und industriellen Produktaufbereitung zum Durchleiten von Flüssigkeiten, zum Einsatz kommt.

### Hintergrund der Erfindung

Die Reinigung von medizinischen Produkten und insbesondere von Hohlraumprodukten in Reinigungs- und Desinfektionsmaschinen nimmt an Bedeutung zu, da sich auch die Operationstechniken immer mehr in Richtung minimal invasive Techniken entwickeln. Es zeigt sich, dass die Hohlraumreinigung in den Maschinen an Grenzen stößt, da der Kreislauf der Reinigungsflüssigkeiten nicht beliebig steigerbar ist. Es werden daher neue Lösungen und wirtschaftliche Verfahren im Bereich der Medizin-, Labor- und Industriereinigung gesucht, um produktschonend und effektiv Hohlraumprodukte zu reinigen oder Flüssigkeiten zielgerichtet weiterzuleiten.

Stand der Reinigungstechnologie ist es heute, dass Spülleisten und Spülzylinder für die Produktreinigung eingesetzt werden. In DE 10 2004 029 970 B3 werden hierzu Spülleisten und in DE 10 2008 050 991.4 Spülzylinder mit Partikelfilter eingesetzt. Desweiteren werden unterschiedliche Adapter benötigt, um hier Hohlraumprodukte anschließen zu können. Das größte Problem bei der Hohlraumproduktereinigung ergibt sich jedoch dadurch, dass die maschinengesteuerten Systeme viele Öffnungen besitzen und dadurch ein erheblicher Druckverlust resultiert. Die derzeitigen technischen Lösungen stellen Rückschlagventiltechniken dar, die sich nicht für die Hohlraumproduktereinigung eignen. Als bekannte Verschlusstechniken werden Klappenventile, Membranscheiben, Absperrelemente oder Rückschlagventile eingesetzt. Dabei kommen einige Lösungen ohne Einsatz von Federn, Ventilen aus, wie im Patent DE 20 2008 009 245 U1 beschrieben. Diese bestehen aus flexiblen Kunststoffen und besitzen mittig angeordnete Stützen. Andere Techniken hingegen nutzen Klappenventile, die durch eine Feder verschlossen werden. Eine dieser Lösungen ist in DE 296 19 635 U1 für Trokar-Hülsen beschrieben. Alternative neuere Erfindungen, wie in DE 10 2008 006 686 A1, nutzen Rückschlagventile, bei dem der Ventilsitz und die Sperrklappen schräg gestellt sind. Komplizierte Klappenventile, wie in DE 10 2008 062 532 A1 gezeigt, nutzen Absperrelemente, die einen Beeinflussungskörper aktivieren, um z. B. die Lageänderung der Klappe aufzuzeigen. Zum weiteren Stand der Technik gehört GB 1 343 903 A1 bei der ein vor- und zurückbewegbares Verschlussteil die Vorrichtung verschließt und eine bewegbare Verbindung hergestellt werden kann. Ebenfalls ist in US 2 457 052 A ein bewegliches und fixierbares Ventilteil und in GB 1 466 242 A ein zylindrischer Ventilkörper vorgesehen, der durch einen Stift verschließbar ist.

Bei den bekannten technischen Ausgestaltungen werden in der Regel komplizierte und teuere Lösungen eingesetzt mit dem Ziel, einen nahezu vollständigen Verschluss zu erzielen. Es werden jedoch Verschluss- und Klappentechniken im Bereich der Reinigungs- und Implantat-Techniken gesucht, die zuverlässig, kostengünstig und einfach einsetzbar sein müssen. Darüber werden Verfahren gesucht, die einen zu großen Druckabfall in Flüssigkeitskreisläufen vermeiden helfen. Dabei muss der Verschluss nicht im Hochdruckbereich funktionieren. In der Medizintechnik ist es vom allgemeinen Interesse, dass bei Durchspülung von Produktkanälen und der Reinigung von Instrumenten diese mit ausreichenden Reinigungsmedien durchströmt werden können, sicher gehalten und enge Lumen nicht von Partikeln verunreinigt werden. Besonders nachteilig ist derzeit, dass im Bereich der Reinigungs-Maschinentechnologien mit offenen Systemen gearbeitet wird und durch den Flüssigkeitsausstrom starke Druckschwankungen entstehen können, was wiederum die Hohlraumproduktereinigung und die Validierung der Prozesse erschwert. Zwar werden bereits verschließbare Kopplungsadapter um den Druckabfall einzuschränken, doch diese Ausgestaltungen ermöglichen keine direkte Produkteinigung z. B. von Instrumentenspitzen o. dgl..

### Zusammenfassung der Erfindung

Die Erfindung hat die Aufgabe das Spül-, Kopplungs- und Anschlussproblem insbesondere im Bereich der medizinischen Produktreinigung zu lösen und dabei flexible Spülmodule in Siebkörben und ein Spülverfahren in Verbindung mit Reinigungsmaschinen zu ermöglichen. Eine weitere Aufgabe der Erfindung ist einen geeigneten Verschluss und eine unkomplizierte vielseitige Produktkopplung beim Spülen, Anschließen und/oder Halten oder Reinigen von Produkten insbesondere im Bereich der medizinischen Instrumentenaufbereitung oder der industriellen Anwendung bereit zu stellen. Ziel ist, die Produktaufbereitung durch eine modular aufgebaute verschließbare Spül- und Kopplungsvorrichtung, auch in Verbindung mit einer integrierten Produkthalterung, zu unterstützen. Dabei soll die Vorrichtung sterilisierbar und so weit erforderlich auch für kleine Instrumente nutzbar sein. Ferner ist eine modular aufgebaute Vorrichtung vorgesehen, welche mit einem Rohrverteiler einer Spülleisten, einem Spülzylinder oder ähnlichen Vorrichtungen lös- und änderbar verbunden werden kann. Weiterführende Anwendungen der Technologie sind Venen-Implantate, um den Rückfluss z. B. von Blut wie bei einer Taschenklappe zu unterbinden, oder im Bereich der Deckeltechnologie denkbar. Die Ausgestaltung der Erfindung ist damit nicht nur auf Anwendungsbereiche im Bereich der Reinigungs- und Desinfektionsmaschinen beschränkt, sondern auch in der Medizin und für andere industrielle Bereiche, z. B. im Bereich der Lebensmitteltechnologie als Auslaufschutz in Tuben etc. oder im Bereich der Labortechnik als Deckelsystem, nutzbar.

Diese Aufgabe wird erfindungsgemäß durch eine verschließbare Vorrichtung zum Spülen, Anschließen, Koppeln und/oder Halten oder Reinigen von Produkten, die insbesondere in Reinigungsmaschen in der Medizin-, Labor- und Industrietechnik Anwendung findet, durch Anspruch 1 und durch ein Spülverfahren nach Anspruch 11 gelöst. Folgende Ansprüche betreffen die vorteilhaften Ausgestaltungen der Erfindung.

Die Neuerung löst die Aufgabe dann sinnvoll, wenn eine verschließbare Vorrichtung zum Spülen, Anschließen, Koppeln und/oder Halten oder Reinigen insbesondere im Bereich der medizinischen und industriellen Produktaufbereitung zum Durchleiten von Flüssigkeiten, Lösungen, biologischen Flüssigkeiten oder zähfließender Stoffen zum Einsatz kommt und dadurch gekennzeichnet ist, dass unter einem Anschlags-, Dicht- und/oder Halteelement mindestens ein vor- und zurückbewegbares Klappen-, Bürsten- oder Fächerelement angeordnet ist, um diese durch einbringen eines Rohrverteilers, eines Adapters, ein zu reinigendes Produktes oder dergleichen nach unten in einen Kanal oder Raum zu drücken und dadurch zumindest eine bewegbare Verbindung zur Flüssigkeitsweiterleitung und/oder Produktreinigung in der Vorrichtung herzustellen.

Die Ausgestaltung der Erfindung sieht dabei vor, dass das Anschlags-, Dicht- und/oder Halteelement zumindest eine Lochscheibe, eine Kappe oder ein Drahtelement ist und einen Öffnungsbereich besitzt. Durch diese Öffnungen ist es möglich unterschiedliche Produktgeometrien und Produktquerschnitte in die Vorrichtung einzubringen. Nach der Entnahme werden die beweglichen Teile der Vorrichtung (Klappen, Bürsten, Fächerscheiben o. dgl.) zumindest wieder durch den Flüssigkeitsstrom zurückbewegt und an diese Anschlagsbegrenzung anliegen.

Durch den Einsatz von elastischen Halte- und Dichtelemente z. B. aus Silikon als Anschlagsbegrenzung, bleiben die eingebrachten Produkte in der Vorrichtung zumindest drehbar und können je nach Ausführung der verschließbaren Vorrichtung auch seitlich in mehreren Richtungen sowie vor- und zurückbewegt werden.

Die Neuerung sieht weiter eine flexible und modular einsetzbare und verschließbare Produktkopplungsvorrichtung vor. In diese Vorrichtung können Adapter mit einem vorderen Kopplungsteil und zumindest einer Halterungsnute und die in Weiterbildung Luer Lock oder Schlauch-Elemente, Spüllanzen o. dgl. besitzen, eingebracht werden, ohne das dabei mit Federn oder ähnlichen Teilen eine ausschließliche kraftschlüssige Verbindungen hergestellt wird. Weitere Anschlussmöglichkeiten, zumindest für medizinische Instrumente, Schläuche, Halterungsblöcke o. dgl. können so entstehen und lösbar miteinander lösbar verbunden werden. Eine elastische und bewegbare Verbindung wird dabei, z. B. durch eine Halterungsnute ermöglicht, in der der Öffnungsrand einer Kappe oder einer Scheibe eingebracht wird und es dabei zu einer kraft- und/oder formschlüssigen sowie elastischen Verbindung kommt. Erst durch Überwindung einer ausreichenden Rückstellkraft bzw. Zugkraft wird diese zumindest formschlüssige Verbindung, gelöst. Diese Art der Verbindung ist besonders flexibel und viel einfacher zu handhaben als bekannte Kopplungsvorrichtungen mit starren Elementen und anfälligen Federverschlüssen.

Die angesprochenen Fächerscheiben besitzen vorzugsweise überlappende Flächen oder Fächer. Dadurch dichten die Scheiben und unterbinden oder behindern zum Teil den Flüssigkeitsausstrom. Bürsten oder Fächerscheiben haben den Vorteil, dass sich diese in der Regel auch von alleine und nicht nur durch den Flüssigkeitsstrom schließen oder zurückbewegen können.

Ziel der verschließbaren Vorrichtung insbesondere auch zum Reinigen von medizinischen Produkten ist, dass ein zu großer Druckabfall in offenen Flüssigkeitskreisläufen von Reinigungsmaschinen vermieden wird und ein konstanterer Reinigungserfolg erzielt werden kann. Durch Einbringen bzw. Eindrücken eines Hohlraumproduktes oder eines Rohrverteilers, z. B. über die obere Lochscheibe, Lochkappe oder Stopfen, wird hierdurch eine Klappe geöffnet. Diese bleibt so lange offen, bis das Hohlraumprodukt wieder entfernt wird. Dadurch wird eine Durchleitung von Medien über mindestens einen Produktkanal des eingebrachten Produktes oder Rohrverteilers ermöglicht. Wird das Produkt wieder entfernt, schließt sich die Klappe durch den Flüssigkeitsrückfluss bzw. -ausstrom und legt sich wieder an die Anschlagsbegrenzung an. Die Klappe öffnet sich aber auch durch ein einströmendes Medium, wenn dieses z. B. über die Öffnung in der Kappe oder einer Lochscheibe erfolgt.

Die verschließbare Vorrichtung kann dabei auch mit einem Filter, Druckminderer oder mit Abdeckungen mit mehreren Durchbrüchen kombiniert sein. Halterungsblöcke auch mit auswechselbaren Rohrverteilern bilden eine erweiterte Vorrichtung. Dabei kann der Block zumindest seitlich verschließbar sein. Halterungsblöcke haben den weiteren Vorteil, dass diese links und/oder rechts Rohrverteiler aufnehmen oder über Zusatzvorrichtung mit der Siebkorbwand lösbar verbunden werden können oder im Siebkorb lose liegen. Letztere Zusatzvorrichtung kann über eine Klemmung oder durch eine Schraubverbindung erfolgen. Diese Vorrichtung vereinfacht und erweitert die Anschlussvielfalt in einem Reinigungsmaschinenwagen oder einem Siebkorb. Einfachere und verschließbare Vorrichtungen in Form von Anschlussadaptern, Rohrverteilern oder Spülleisten lassen sich dann besonders wirtschaftlich einsetzen, wenn diese mobil und modular aufgebaut und vorzugsweise in Siebkörbe gelegt werden.

Verschließbare Vorrichtungen mit einem Klappenverschluss in Verbindung mit Maschinen werden dabei bevorzugt als Verschlussteil auf Rohrverteiler, Rohrenden oder als Einbauteil in Spülzylinder eingesetzt. Alternativ kann vorgesehen sein, dass die Vorrichtung als ein röhrenförmiges Implantat eine biologische Vene oder Arterie - sprich Gefäße - verbindet. Vorzugsweise wird diese im Anschlussbereich vernäht oder hier durch einen Stent verbunden.

Vorzugsweise bestehen die beweglichen Teile der Vorrichtung aus Bürsten, Scheiben- oder Klappenteile aus einem elastischen Kunststoff. Alternativ können alle Teile der Vorrichtung aus Kunststoff bestehen und unterschiedliche Lochgeometrien oder Durchbrüche aufweisen die auch übereinander liegen oder sich dabei überlappen können. Von Vorteil sind Scheiben als Lochblenden oder Kappen mit einer Öffnung im Deckel als Anschlagsbegrenzung. Um einen sicheren Verschluss der Lochscheiben oder der Lochkappen zu ermöglichen, kann die anliegende Klappe einen erhöhten Materialrand ähnlich einem Tellerrand oder eine Tasche oder eine zusätzlich aufgelagerte Klappe aufweisen und bei vorliegen mehrerer Klappen sich diese dabei auch überlagern. Dieser erhöhte, auch umlaufende Rand einer Klappe oder eine vorhandene Tasche verbessert den Verschluss der Klappe. Durch den Rück- bzw. Ausstrom wird so mehr Medium eingefangen, um diese zu schließen. Eine und/oder mehrere Taschen oder Segel oder Materialerhöhungen zumindest auf der Klappenfläche, funktionieren dabei wie eine Oberflächenvergrößerung oder ermöglichen wie ein Federteil eine Rückstellkraft. Alternativ können hier Noppen auf der Oberfläche angeordnet sein, um die Produktreinigung zu verbessern und Spülschatten zu reduzieren. So wird z. B. verhindert, dass sich die Klappe vollständige an eine Wand anlegt. Derartige konstruierte Klappen mit Taschen, Klappensegel mit Noppen oder einer Randstruktur verbessern die Verschlusssicherheit und Funktion der Klappe auch bei niedrigen Flussgeschwindigkeiten. Die Herstellung der Klappen kann bei Scheiben einfacher weise durch Ausstanzen erfolgen, aber auch als Spritzteil oder Gießformteil hergestellt werden.

Es ist vorgesehen, dass die flüssigen aber auch zähfließenden Medien über mindestens einen eingebrachten Produktkanal nach außen geleitet werden, ohne dass zu viel Flüssigkeit seitlich am Produkt vorbeiströmt. Die Klappe hat aber noch eine Funktion, sie hält durch eine seitlich anliegende Anpresskraft das Produkt besser in der Verschlussvorrichtung. Dieser Haltemechanismus wird verstärkt durch mehrere Klappen oder durch Materialverstärkungen, Materialerhöhungen, Taschen oder Segel auf der Klappenoberfläche, weil diese die Schließkraft durch die zurückfließende oder ausströmende Flüssigkeit erhöht und zusätzlich auch als Abstandshalter dient. Damit wird das eingebrachte Hohlraumprodukt in einem Spülzylinder oder einen Rohrverteiler nicht so leicht über den Medienstrom herausgedrückt.

Der Aufbau der Neuerung kann so gestaltet sein, dass mindestens ein Bürstenelement, ein Kappenelement oder eine Scheibe oder mehrere Kappen und/oder Scheiben, die übereinanderliegen, einen Medienverschluss an einem Rohrverteiler ermöglichen. Dabei kann eine elastische Lochkappe durch überstülpen von oben eine untere Scheibe mit einer Klappe halten oder ein Deckel mit einem Durchbruch ein Bürsten- oder Fächerelement oder eine Lochscheibe über eine Kappe mit einer Klappe fixieren. Alternativ hierzu kann auch ein Bürsten- oder ein Fächerelement mit einer unteren integrierten Klappe oder einer Scheibe mit einer Klappe zum Einsatz kommen und diese Klappe nach oben begrenzen. In Weiterbildung kann die Klappe auch durch einen anderen oder zusätzlichen geeigneten auch starren Niederhalter in Form einer Klappenanschlagsbegrenzungseinheit in Beweglichkeit nach begrenzt sein. Diese Klappenanschlagsbegrenzung wiederum kann so gestaltet sein, dass hiermit auch ein Produkt zusätzlichen Halt findet oder ein Hohlraumprodukt so gehalten wird, dass Flüssigkeit besser durch den inneren Kanal strömen kann. Vorzugsweise kommen dabei Klappenanschlagsbegrenzungen in Form einer Drahtvorrichtung oder als Rohrelement in Frage, die über z. B. einer Kappe mit einer Klappe gestülpt wird.

Um einen Verschluss der Vorrichtung zu erreichen, sollte z. B. eine bewegliche Klappe und ein geeignet großer Anschlussraum oder Austrittskanal vorhanden sein die sich diese hinein bewegen kann. Dadurch, dass die aufliegende Durchbruchsfläche einen kleineren Durchmesser als die untere Klappenfläche aufweist, wird die Klappe am überstehenden Flächenteil anliegen und so den Durchbruch verschließen. Die Neuerung besitzt gegenüber bekannten Spülvorrichtungen den besonderen Vorteil, dass sich die Vorrichtungsteile einfach und individuell austauschen oder anordnen lassen. Beispielsweise können kostengünstig Lochscheiben oder Lochkappen als Klappenanschlagsbegrenzung ausgetauscht werden. Damit wird sichergestellt oder ist es einfach möglich, dass signifikant unterschiedliche Instrumentenquerschnitte mit einer Klappenvorrichtung so gedichtet werden, dass die Flüssigkeit durch den Produktkanal strömt und nicht der größte Teil am Produktkanal vorbeigeleitet wird.

Ein besonderer wirtschaftlicher Vorteil der Erfindung ist, dass die Vorrichtungsteile sehr kostengünstig hergestellt werden können. Dies ist besonders bei der Verwendung von Kappen gegeben, wobei zumindest die untere Kappe eine Klappe aufweist und durch eine überstülpte Kappe mit einer geeigneten Öffnung als Anschlagsbegrenzung die erfinderische Vorrichtung bildet. Besitzen dabei die Kappen einen Abstand zu einem Rohrverteiler oder einem Adapaterteil, so entsteht eine besonders vielseitige Vorrichtung in der hier eingebrachte Produkte sowie Kopplungsteile drehbar und auch seitliche bewegt werden können. Dies verbessert die Vielseitigkeit von Kopplungsteilen zur Flüssigkeitsweiterleitung und vereinfacht bei der Produktreinigung z. B. das zusammenbringen von Spüllanzen die in einen Produktkanal eingeführt werden müssen um eine ausreichende Produktreinigung zu erzielen. Diese hohe Flexibilität der Vorrichtungsteile wird in der Form einer einfachen Lochscheibe oder Lochkappe noch erhöht, in dem über z. B. einer zusätzlichen Verlängerungshülse eine Instrumentenhalterung oder durch Einbringen von Lochscheiben oder Filterscheiben in einer Anschlussvorrichtung in Ausbildung eines Spülzylinders (wie in Patent DE 10 2008 050 991.4 Filter und Aufbereitungsvorrichtung beschrieben) eine zusätzliche Partikelfilterung möglich wird. Alternativ kann auch eine Filterscheibe oder eine Filtermodul zwischen den Kappen oder Scheiben platziert sein und diese nur als Filter dienen. Alternativ kann ein verschließbarer Durchlass (Scheiben und/oder Kappen) auch in einem Dreharm eingebracht sein, mit dem Ziel, Flüssigkeit zu filtern und/oder Flüssigkeit zu verteilen. Dabei sind die Klappen so konstruiert, dass diese den Wasserausstrom durch das Klappendesign besser verteilen und gleichzeitig filtern. Vorzugsweise kommen hier Mehrfachklappen, die durch einen Kreuzschlitz oder in ähnlichen Schnittmustern hergestellt wurden, zum Einsatz. Diese wiederum können dabei über einen Niederhalter, der gleichzeitig auch Fixierungsteil sein kann, gehalten werden.

Wird die Neuerung in Form eines Verbindungselementes endständig mit einem Gefäßimplantat verbunden, so ermöglicht diese Weiterbildung der Erfindung eine Venenklappe oder ein Klappenunterstützungssystem in Arterien (z. B. Aorta). Wird die Klappe dabei aus einem Kollagenmischgewebe oder ähnlichem ausgeführt, so kann die Klappe mit Endotehlzellen besiedelt werden. Dabei kann die Besiedlung auch direkt im Körper erfolgen, weil bekannt ist, dass ein Endothelzellbewuchs einige Millimeter im Einstrombereich von künstlichen Gefäßprothesen erfolgt.

In Weiterbildung der Erfindung sind auswechselbare Produkthalterungen auch als Klappenbegrenzungshalter vorgesehen, die mit der Klappenverschlussvorrichtung kombinierbar sind. Dabei kann einfacher Weise, ein von außen angebrachtes Halterungsrohr mit Durchbrüchen vorgesehen sein. Durch Kombination mit einem stabilen weiteren Halterungsteil wird ein Hohlraumprodukt zusätzlich gehalten. Das Halterungsteil kann dabei sehr lang ausgeführt sein, um lange Hohlrauminstrumente zu halten. Um besonders schwierige und kleine Instrumententeile, wie Bohrer, zu reinigen, kann vorgesehen sein, dass die Lochscheibe oder die Lochkappe mehrere Öffnungen oder Öffnungsgrößen besitzt. Da die Scheiben oder Kappen einfach austauschbar sind, wird eine effiziente und wirtschaftliche Reinigung dieser englumigen Hohlkörpern sowie von Bohrern vereinfacht.

In Weiterführung der Erfindung kann auch vorgesehen sein, dass über die Vorrichtung Prüfkörpervorrichtungen eingesteckt werden. Dadurch, dass die Vorrichtung teilbar ist, kann der Testkörper auch ein Filterelement sein. Das Element wird dabei zwischen den Scheiben oder den Kappen platziert.

Ein anderer Ansatz kann aber auch in der Art gestaltet sein, dass z. B. eine Lochkappe ein Bürsten- oder Fächerelement fixiert. Diese Elemente haben den Vorteil, dass hier ein eingebrachtes Produkt durch die sich anliegenden Bürsten oder Fächerelemente gehalten wird. Ferner streifen die Bürsten und Fächer Verunreinigungen an den Außenflächen der zu spülenden Produkte ab und verhindern zusätzlich, dass zu viel Flüssigkeit außen am Instrumentenschaft vorbeiströmt. Diese Ausgestaltung hat den weiteren Vorteil, dass die Büsten oder Fächer sehr flexibel sind und damit unterschiedliche Produktquerschnitte in der Vorrichtung eingebracht werden können.

Die verschließbare Vorrichtung hat ferner zum Ziel zu starke Druckschwankungen in Reinigungsmaschinen zu verhindern und die Produktaufbereitung so sicher zu gestalten. Zur Lösung dieses Problems wird ein Spülverfahren in Verbindung mit Flüssigkeits- oder Lösungskreisläufen, dass insbesondere in Reinigungsmaschen in der Medizin-, Labor- und Industrietechnik Anwendung findet, eingesetzt und dadurch gekennzeichnet ist, dass in einem Flüssigkeitskreislauf zumindest ein Spülplatz oder ein Spülanschluss zumindest eine verschließbare Vorrichtung besitzt, die sich durch mindestens eine Klappe oder über Bürsten oder Fächerscheiben durch den Flüssigkeitsstrom oder von alleine schließt und eine Produktspülung erfolgt, wenn dieser Spülplatz oder Spülanschluss zumindest durch einen eingebrachten Kanal oder ein Produkt belegt ist und eine reduzierte Flüssigkeitsmenge oder keine Flüssigkeit austritt wenn dieser nicht belegt ist.

Spülplätze können dabei Anschlussadapter, Rohrverteiler mit mehreren Anschlussadaptern, Rohrverteilerblöcke, Spülrohre, Spülzylinder oder Anschlusszylinder zum Anschließen von Rohrverteiler und/oder direkt zum Durchspülen von Hohlraumprodukten sein. Diese können fest oder lösbar miteinander kombinierbar sein. Darüber hinaus fest mit einem Hauptmedienverteiler oder mobil, lose, beweglich oder fest in einem Siebkorb oder ähnlichem oder in einer Maschine bzw. Maschinenwagen eingebracht sein. Das Verfahren wird dabei bevorzugt in Reinigungsmaschinen oder in Ultraschallbädern mit einem Flüssigkeitskreislauf angewendet. Dabei wird die Reinigungsflüssigkeit in einem Behandlungsraum, z. B. über Dreharme, Rohrverteiler oder Sprühdüsen, eingebracht.

Damit ist ein Verfahren möglich, bei dem die die Anzahl der eingebrachten Hohlrauminstrumente variabel gehalten werden kann, ohne die Produktreinigung zu stark zu beeinträchtigen. Weitere Vorteile ergeben sich dadurch, dass signifikant mehr Spülplätze mit Rohrverteilern verbunden sein können als in bekannten Maschinenwagen von Reinigungsmaschinen, da ein zu starker Druckabfall vermieden wird. Eine ausschließliche Verwendung verschließbarer Spülplätze erhöht an jedem Spülplatz die Sicherheit der Produktreinigung in der Form, da hiermit genügend Reinigungsflüssigkeit und ein genügend hoher Reinigungsdruck zur Verfügung steht. Ferner ist der Spüldruck unmittelbar am Spüladapter besser beherrsch- und steuerbar. Diese Beherrschbarkeit und Beeinflussung ist wichtig, weil die Flüssigkeitszuführung über Leitungen in einem Rohrverteiler durch das Leitungslumen begrenzt ist und z. B. die Pumpe in einer Reinigungsmaschine nicht gezielt an dieser Stelle den Druck im Rohrverteiler erhöhen kann. Da in Reinigungsmaschinen die Spüldrücke unmittel bar an Spülplätzen von 0,1 bis 1 Bar oder mehr schwanken können, wird so sichergestellt, dass die Spüldruckschwankung signifikant geringer ausfällt. Letztere Druckschwankungen sind insbesondere vom Hauptverteilungsrohr entfernten Rohrverteilern mit mehreren Ausgängen zu beobachten, wenn der Flüssigkeitszufluss nicht angepasst werden kann. Wünschenswert sind hier Druckschwankungen die 0,5 Bar nicht überschreiten.

Der Einsatz von geeigneten verschließbaren Vorrichtungen, beispielsweise mit elastischen Klappen oder Bürsten oder Fächerscheiben, hat den weiteren Vorteil, dass der Flüssigkeitsstrom in bestimmten Grenzen zusätzlich gepuffert wird.

Somit ist das Ziel der Erfindung, den Druck in einem offenen Flüssigkeitslauf einer Reinigungsmaschine so konstant wie möglich zu halten und/oder den Ausstrom an den Spülplätzen zu begrenzen und zu beeinflussen und in einem geschlossenen System den Rückstrom von Flüssigkeiten, z. B. in elastischen Rohrleitungen bzw. Gefäßen, über verschließbare Vorrichtungen zu puffern und zu unterbinden.

Weitere Vorteile ergeben sich, wenn dass Spülverfahren mit modularen Vorrichtungen betrieben wird. Für die Hauptmedienversorgung von Rohrverteilern kann beispielsweise ein Halterungsblock oder der Anschlusszylinder Anwendung finden, die miteinander kombinierbar sind. Dabei ist vorgesehen, dass insbesondere an den Anschlussadaptern eine Klappe oder ähnliches durch das eingebrachte Produkt oder Rohrverteiler geöffnet wird. Nach Entnahme der Rohrverteiler oder der Produkte schließt sich die Klappe durch den Flüssigkeitstrom wieder. Alternativ kann hier auch nur ein Bürstenelement oder überdeckende Fächerscheiben zum Einsatz kommen, um einen definierten Ausstromwiderstand zu ermöglichen. Damit wird ein zu großer Druckverlust im Flüssigkeitskreislauf verhindert und über Rohrverteiler können insbesondere mehrere medizinische Hohlrauminstrumente umund/oder durchströmt werden. Darüber hinaus wird die Validierarkeit von Reinigungs- und Desinfektionsmaschinen sowie von Spül- und Reinigungsverfahren insgesamt verbessert und es ist nicht mehr so wichtig, wo und wie viele zu reinigenden Hohlraumprodukte an den Rohrverteilern angeschlossen sind. Beim alten Stand der Technik ist es nämlich so, dass voll bestückte Rohrverteilerleisten ein besseres Reinigungsergebnis erzielen, als wenn diese nur halb bestückt sind, da durch die nicht verschlossenen oder nicht genutzten Öffnungen zu viel Flüssigkeit herausströmt. Der Reinigungserfolg alter Technologien ist damit mehr oder minder von der Art der Hohlraumproduktbestückung abhängig.

Damit erlaubt die Erfindung ein neues Verfahren zur Aufbereitung von Hohlraumprodukten in der Art, dass der Reinigungsdruck auch von Flüssigkeit die in längeren Leitungen bewegt wird, zumindest beim Einssatz von Rohrverteilern mit mehreren Anschlüssen oder von Zylindern mit verschließbaren Vorrichtungen sich dadurch verbessert, dass nach der Entnahme oder das herauslösen eines Hohlraumproduktes aus einer ganz oder nur zum Teil verschließbaren Spülvorrichtung der gefürchtete Druckabfall ausbleibt bzw. der Druck nicht abfällt. Es wird sogar so sein, dass durch die Entnahme mehrerer Produkte der Reinigungsdruck steigen kann und damit der Reinigungserfolg zumindest in einer Reinigungsmaschine verbessert wird. Hohlraumprodukte die sich an Spülplätzen von Rohrverteilern befinden, können durch den Reinigungsdruck in einer Reinigungsmaschine herausgeschoben werden. Dieses muss verhindern werden, da ansonsten sich der Druck im Flüssigkeitskreislauf signifikant ändert bzw. fällt und damit auch das Produkt nicht gereinigt wird. Darüber hinaus wird immer wieder vergessen, geöffnete Spüladapter zu verschließen. Meisten jedoch lassen sich diese nicht schließen. Damit ist das Reinigungsergebnis in einer Reinigungsmaschine nicht mehr so davon abhängig, wie der Maschinenwagen bestückt ist, welche Anzahl offener oder belegter Spülplätze vorhanden sind sowie von der Lage der Spülplätze und mitunter auch von der Sorgfaltspflicht der Person, welche die Bestückung vornimmt.

Die Weiterbildung des Verfahrens ermöglicht es, dass Hohlraumprodukte in einem Siebkorb verbleiben und nicht mehr auf die unterschiedlichsten MIC-Wagen oder Maschinenwagen verteilt werden. Ein Umpacken und Umsortieren des Aufbereitungsgutes im Instrumentenaufbereitungskreislauf wird so reduziert oder ganz verhindert, weil auch die Hohlraumprodukte über eingebrachte Spülplätze in Form eines Rohrverteilers, Zylinders oder von Adaptern in einem Siebkorb oder auf einem Gestell gereinigt werden können.

Bei der medizinischen Instrumentenaufbereitung werden tausende von unterschiedlichen Produkten und Produktquerschnitten aufbereitet. Der Einsatz von verschließbaren Vorrichtungen zur Produktreinigung und Produktkopplung mit geeigneten Adaptern bietet die Möglichkeit, dass diese unterschiedlichen Produkte und Produktquerschnitte durch schnell auswechselbare Produkthalterungen und/oder Produktdichtungen durchspülbar sind. Dies wiederum erhöht das Anwendungsspektrum und die Sicherheit des Verfahrens sowie die Akzeptanz beim Nutzer. Letzteres gilt auch für die Außenreinigung von Produkten, da so mehr Flüssigkeit für die Außenreinigung zur Verfügung steht und nicht aus ungenutzten Spülplätzen ungenutzt herausströmt. Die beschriebene Neuerung besitzt damit ein hohes Kosteneinsparungspotential bei gleichzeitiger Erhöhung der Aufbereitungssicherheit.

### Einsatzgebiete und Ausführungsformen

Es ist vorgesehen, die erfinderische verschließbare Vorrichtung mit mindestens einer Klappe mit Bürsten oder Fächern an oder in Rohrverteilern, Spülleisten, Spülzylindern so zu nutzen, das damit das erfinderische Verfahren ermöglicht wird.

Die Vorrichtung ermöglicht die Reinigung von fast allen Hohlrauminstrumenten. Hierzu gehören Augenkanülen, MIC-Instrumenteile, HNO-Sauger, Saver-Blads, Dentalbohrer oder Dentalhandstücke sowie andere Hohlraumprodukte. Das Einsatzgebiet der Vorrichtung und des Verfahrens ist jedoch nicht nur auf medizinische Produkte beschränkt, sondern läßt sich überall dort einsetzen, wo eine Produktspülung und/oder eine lösbare Produktkopplung z. B. mit Adaptern oder mit Steckverbinder gewünscht wird.

Folgende Anwendungsbereiche sind für die erfinderische verschließbare Vorrichtung vorgesehen:
1. Absperr- und Anschlussvorrichtung an Ausgängen von Rohrverteilern
2. Als Kopplungs- und Verschlussteil für Rohrverteiler an Hauptrohrverteilern von Reinigungs- und Desinfektionsmaschinen
3. Halterungs- und Steckmodul für medizinische Hohlrauminstrumente
4. Halterungen-, Kopplungs- und Steckmodule für bekannte Luer Lock Adapter, Schlauchverbinder oder Spüllanzen die in der Medizintechnik eingesetzt werden
5. Anschlussmodul und Medienverteiler mit Partikelfilterung
6. Abstreifmodul für grobe Verunreinigungen auf Außenflächen
7. Anschlussmodul für Reinigungskontrollen
8. Verschlussteil für Produkte in Verbindung mit einem Rohr
9. Anschluss- und Verbindungsteil für Gewebeimplantate
10. Austrittsvorrichtung an Dreharmen von Reinigungsmaschinen

In bevorzugter Ausgestaltung besitzt die erfinderische Vorrichtung einen runden oder geometrischen Querschnitt aus Kunststoff und besitzt mindestens zwei auch getrennt voneinander nutzbare Teile. Der bevorzugte Kunststoff für die Scheiben und Kappen ist Silikon, da dieser temperaturstabil ist. Dabei dient das obere Vorrichtungsteil als Anschluss oder Produkthalterungsteil. Der untere Vorrichtungsteil enthält die bewegliche Klappe, Bürsten oder Fächerscheiben. Von Vorteil ist, wenn beide Teile aus elastischem Material gefertigt sind, da diese so besser über ein Rohrendstück gestülpt werden können.

Das bevorzugte Klappendesign ist eine flache Klappe, die auch auf der Oberfläche Materialerhebungen z. B. in Form einer Noppe aufweisen kann. Wird die Klappe aus einem Plattenmaterial hergestellt, so können im Schnittbereich kleine Löcher vorhanden sein, um hier ein Einreißen zu vermeiden. Komplizierte Klappen mit zusätzlichen Taschen oder Randerhöhungen werden durch Spritzguß hergestellt und können sehr dünnwandig ausgeführt sein. Werden die Klappen in Flüssigkeitskreisläufen mit Drücken über 0,5 Bar verwendet, kann es von Vorteil sein, wenn die Klappenfläche ein Loch aufweist. Das Loch soll verhindern, dass die Klappe sicher schließt und nicht durch den Druck zu schnell herausgedrückt wird.

Eine technische Ausgestaltung eines Spül- oder Anschlussplatzes wird durch einen runden Zylinder gebildet, der ein Außengewinde besitzt und mit einem Deckel mit einem großen Durchbruch verbunden werden kann. Bei der zylindrische Konstruktion wird zuerst eine Scheibe mit einem beweglichen Klappenteil eingebracht und hierauf eine Lochscheibe aufgelegt. Durch den Deckel werden die Scheiben fixiert. Im Bodenbereich des Zylinders kann eine runde Filtereinheit eingebracht sein. Die obere Lochscheibe kann dabei ein oder mehrere Löcher aufweisen oder auch ein Bürsten- oder Fächerelement. Wird ein elastisches Material eingesetzt, so wird das eingebrachte Produkt oder ein Rohrverteiler im Lochbereich abgedichtet.

Die einfachste Ausgestaltung eines Spül- oder Anschlussplatzes wird aus zwei übereinander gesteckte Kappen gebildet, wovon die unterste Kappe eine Klappe besitzt. Vorzugsweise besitzt die stülpbare Kappe zumindest eine runde und/oder sternförmige Öffnung. Eine kleine zusätzliche Öffnung kann dabei eine bessere Hinterspülung eingebrachter und zu reinigender medizinischer Instrumententeile ermöglichen. Dabei kann diese starr oder elastisch ausgebildet sein. Die Kappen werden dabei einfach über ein Rohrende oder einem Schlauchadapter gestülpt. Dabei kann vorgesehen sein, dass die Kappen über Kabelbinder, Schlauchschellen, Federteile oder ähnliches und/oder über ein weiteres Verlängerungsmodul von außen an die Rohrwand gedrückt werden. Das Verlängerungs- oder Halterungsmodul (z.B. ein Federteil das außen anliegt) könnte vorzugsweise dann sinnvoll sein, wenn hier lange medizinische Instrumente senkrecht eingesteckt werden oder die Kappen eine Formstabilisierung benötigen. Diese Ausgestaltung ermöglicht ferner durch Einbringen weiterer Steckteile, einen Luer Lock Anschluss, einen Schlauchanschluss oder einen Spüllanzenanschluss oder o. dgl.. Damit erhält die Erfindung eine hohe Flexibilität und Modularität. Die Neuerung bildet damit die Basis für eine neuartiges Steckmodul- und Kopplungssystem im Bereich der industriellen Anwendungen zur Weiterleitung von Flüssigkeiten.

Bei einer Einteilversion befindet sich die Klappe in einem Rohrverbinder. Damit die Klappe bevorzugt sich nur in einer Richtung öffnet, müssen hier im Inneren eine Querschnittsverengung sowie eine Querschnittserweiterung vorhanden sein. Ein derartiges Teil ist nur über ein Formteilspritzverfahren herstellbar. Mit einem derartigen Aufbau können Schläuche oder Gefäßprothesen miteinander verbunden werden. Alternativ kann auch nur ein rundes Bürstenmodul mit einer Klemmverbindung über Rohrenden gestülpt sein. Dabei liegen viele Bürsten über und nebeneinander, um einen hinreichen Verschluss für Medien zu erhalten. Diese Art eines Verschlusses lässt immer Flüssigkeiten durch, was auch gewünscht sein kann, da die Bürsten ja auch selber gereinigt werden müssen.

Die bevorzugte Ausgestaltung einer verschließbaren. Vorrichtung bzw. eines Spümodules besteht zumindestens aus einem Rohrverteiler, der über die Länge verteilt zylindrische Ansatzstücke aufweist. Um einen Klappenverschluss zu erreichen, werden hier die Kappen über die zylinderischen Ansatzstücke gestülpt. In Weiterbildung wird zumindest ein Rohrverteiler oder eine Spülleiste in einem Verteilerblock oder einem Spülzylinder gesteckt. Damit die Öffnung im Block oder im Zylinder sich wieder von selbst verschließt, ist hier ein Klappenverschluss aus zumindestens einer Scheibe mit einer beweglichen Klappe vorgesehen. Zur besseren Dichtung kann wiederum eine Lochklappe über den Zylinderschaft gestülpt sein und so die eingelagerte Scheibe fixieren. Dabei kann die Vorrichtung über einen Schlauchanschluss und einer Schlauchleitung, aber auch direkt mit einem Hauptmedienverteiler verbunden sein.

Die bevorzugte Ausgestaltung einer Kopplungsvorrichtung an Rohrverteilern wird durch einen Schraubadapter mit einem Schlauchansatz und einem vergrößerten inneren Kanal ermöglicht in der sich hinein die Klappe öffnen kann. Dieser Adapter ist vorzugsweise lösbar mit einem Rohrverteiler verbunden. An dem Schraubadapter befinden sich dabei zwei übereinander gestülpte Kappen, wovon die untere Kappe eine Klappe und die obere Kappe einen Öffnungsbereich besitzt. Zur besseren Fixierung können die Kappen über eine Schlauchschelle am Adapter fixiert sein. In Weiterbildung werden zur Kopplung Adapter mit einer Kopplungsnute eingesetzt. Letztere Adapter ermöglichen weitergehende Anschlussmöglichkeiten, um Lösung aus dem Rohrverteiler weiter zu leiten. Abgeschrägte Adapterteile die in den Rohrverteiler ragen können, sorgen dabei für einen verbesserten Lösungseinstrom in die Vorrichtung.

Weitere Vorteile der Erfindung ergeben sich aus der Wahl der Produkte, die aufbereitet werden sollen sowie der kombinierten Bauteile und Materialien sowie aus der Beschreibung und den beigefügten Zeichnungen.

Es versteht sich, dass die vorstehenden genannten und die nachstehenden noch zu erläuternden Merkmale nicht nur in der jeweils angegebenen Kombination, sondern zum Teil auch in anderen Kombinationen oder in Alleinstellungen verwendbar sind.

Ausführungsbeispiele der Erfindung sind in den Zeichnungen dargestellt und werden in den nachfolgenden Beschreibungen näher erläutert.

Es zeigen:

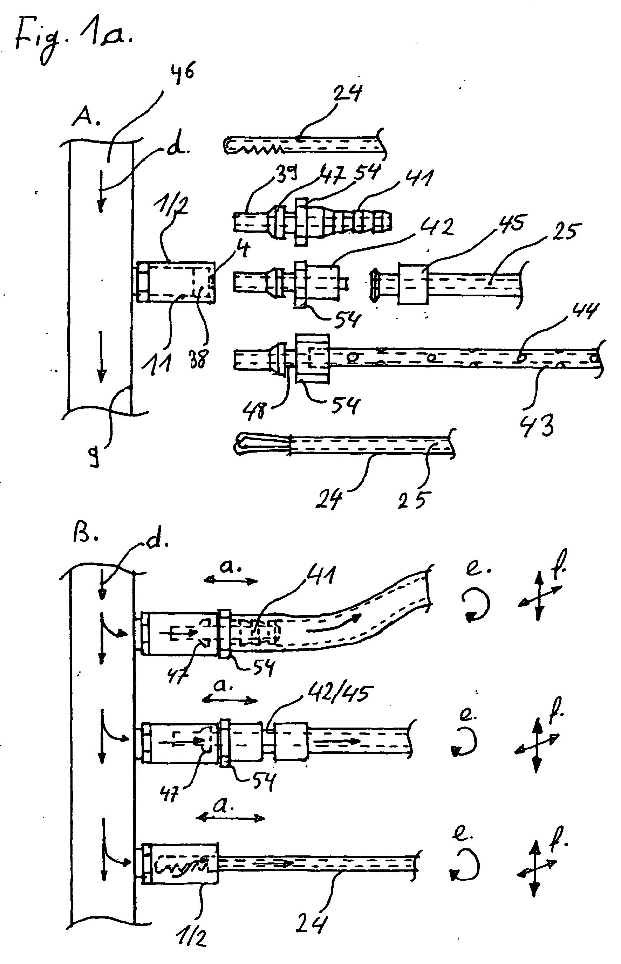

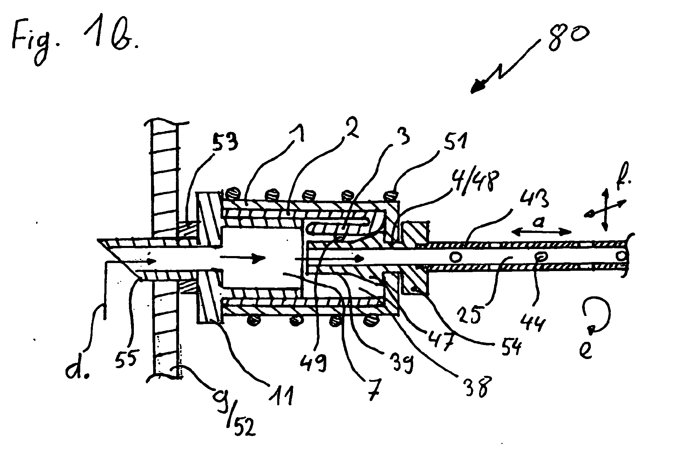

- Fig. 1 a.: Kopplungsdarstellung unterschiedlicher Produktgeometrien und Produktfor- men sowie die Beweglichkeit eingesteckter Produkte und Adaptern in Verbin- dung mit der erfinderischen verschließbaren Vorrichtung.
- Fig. 1 b.: Schnittbild einer erfinderischen verschließbaren Vorrichtung als Kopplungs- modul in Verbindung mit einer Spüllanze.
- Fig. 2a.: Schnittdarstellung einer erfinderischen Spülvorrichtung im geöffneten Zustand in Verbindung mit einem Rohrende.
- Fig. 2b.: Draufsicht eines Spülplatzes oder Spülanschlusses mit einer Spülvorrichtung in Verbindung mit einem Klappenanschlag.
- Fig. 2c.: Draufsicht eines Spülplatzes oder Spülanschlusses mit einem Büstenelement.
- Fig. 3.: Schnittdarstellung einer erfinderischen Spülvorrichtung im geöffneten Zustand in Verbindung mit einem Zylinder.
- Fig. 4a: Draufsicht einer Lochscheibe und einer Scheibe mit einer beweglichen Klap- pe.
- Fig. 4b.: Draufsichten unterschiedlicher Klappendesigns.
- Fig. 4c.: Schnittdarstellung einer Klappe mit einer taschenartigen Erhebung ohne und mit einem Rohrende oder Zylinder.
- Fig. 5: Schnittdarstellung eines Rohr- oder Schlauchverbinders mit integrierter Klap- pe.
- Fig. 6.: Schnittdarstellung einer modular aufgebauten Spülvorrichtung mit unter- schiedlichen Klappenverschluss- und Produkthaltevorrichtungen.
- Fig. 7.: Seitenansicht eines Spülplatzes für medizinische Instrumente mit einer zu- sätzlichen Instrumentenhalterung.

Figur 1a. zeigt eine Kopplungsdarstellung unterschiedlicher Produktgeometrien und Produktformen sowie die Beweglichkeit eingesteckter Produkte und Adaptern in Verbindung mit der erfinderischen verschließbaren Vorrichtung. In Darstellung A ist eine verschließbare Vorrichtung dargestellt die aus zwei Kappen 1 und 2 und einem Adapter 11 gebildet wird, wovon eine untere Kappe 2 eine innere bewegbare Klappe aufweist und die übergestülpte Kappe 1 eine Öffnung **4** besitzt. Diese sind mit einem Adapter, Kopplungsteil oder einem Rohrende **11** mit einem Rohrverteiler **9,** der einem großen Kanal **46** aufweist, Teil einer Maschine oder eine Spülleiste. Dabei besitzt die verschließbare Vorrichtung einen vorderen Raum **38** in die die Klappe bewegt werden kann. Da hier die Seiten aus einem elastischen Material über die Kappen **1** und **2** ausgebildet sind, besitzt diese verschließbare Vorrichtung eine hohe seitliche Beweglichkeit und Flexibilität bei der Kopplung bzw. Verbindung mit eingeführten Produkten **24,** Steckmodulen **54** mit Kopplungselementen **39, 47,48** und Adaptern **41, 42, 43.** Diese können Schlauchverbinderteile **41,** Luer Lock männliche **42** und/oder weibliche Adapterteile **45** oder eine Kombination aus beiden, eine Spüllanze **43** mit Austrittsöffnungen **44** oder ein zu reinigendes medizinisches Instrument **24** mit unterschiedlichen Spitzengeometrien sein. Alternativ wiederum gleichartige und ungleichartig aufgebaute Adapter Kopplungen ermöglichen, insofern diese Kopplungsadapter z. B. als Steckmodul **54** geeignete Kopplungsteile **(39, 47, 48)** besitzen. Um einen sichere Kopplung mit Adaptern zu gewährleisten haben diese Steckmodule **54** ein vorderes Steckteil **39** mit einer Stecksicherung **47** und einer Nut **48** die mit der Öffnung **4** bzw. dem Öffnungsrand der oberen Klappe **1** eine lösbare zumindest Formschlüssige Verbindung herstellt. Ist die Einstecköffnung **4** der Kappe **1** klein wird diese Verbindung zusätzlich auch Kraftschlüssig durch den Öffnungsrand gehalten. In der Darstellung B ist ein medizinisches Instrument **24** bzw. sind Produkte sowie Luer Lock Verbindungsteile **42** und **45** und ein Schlauchverbinderteil **41** mit einem Schlauch **28** verbunden und in die verschließbare Vorrichtung eingebracht. Erst durch das Einbringen der Steckadapter **54** mit den Verbindungsteilen **41, 42** sowie eine Instrumentenspitze eines Instrumentes **24** öffnet sich im inneren der Vorrichtung ein oder mehrere Klappen und ermöglicht so eine Produktreinigung zumindest des vorderen eingebrachten Instrumentteils sowie eine Weiterleitung von Flüssigkeiten (siehe **Pfeil d).** Dabei wird ferner Flüssigkeit durch die Kanäle **25** eines Produktes aus oder über Adapterkanäle weitergeleitet. Diese kann dazu genützt werden, um die Inneren Kanäle **25** eines Instruments zu reinigen. Weiter ist durch die **Pfeile a, e** und **f** angedeutet, dass sich die eingebrachten Produkte **24** und Steckadapter **54** o. dgl. vor- und zurück (angedeutet durch **Pfeil a),** in der Vorrichtung drehbar (angedeutet durch **Pfeil e)** und seitlich in mehreren Richtungen bewegbar sind (angedeutet durch **Pfeil f).**

Figur 1b. zeigt das Schnittbild einer erfinderischen verschließbaren Vorrichtung als Kopplungsmodul in Verbindung mit einer Spüllanze. Mit 80 ist eine verschließbare Vorrichtung in Ausbildung eines Adapters 11 zusammen mit einem Steckmodul 54 im Schnitt dargestellt. Die verschließbare Vorrichtung in Ausgestaltung eines Adapters 11 mit zwei Kappen 1 und 2 sowie einer Haltefeder 51 ist mit einer Wand 52 eines Rohrverteilers beispielsweise in einer Maschine verbunden. Der Adapter **11** ist verschraubt und mit einer zusätzlichen Mutter **53** gesichert und besitzt einen inneren Kanal **7.** Dabei bildet die Feder **51** ein äußeres Korsett, um eine zu starke seitliche Aufweitung der Konstruktion zu unterbinden und die Kappen 1 und **2** mechanisch stärker an die Adapterwand zu drücken. Die innen liegende Kappe **2** weist eine einteilige Klappe **3** mit einer Noppe **49** und die außen liegende Kappe **2** besitzt eine Öffnung **4.** Anstelle einer Klappe kann auch ein mehrteiliges Klappendesign mit mehreren Klappen eingesetzt werden. Über die Öffnung **4** ist ein Kopplungsadapter als Steckmodul **54** in die Vorrichtung eingebracht und die Klappe **3** in den inneren Raum **38** der Vorrichtung hineingedrückt. Die drehbare und bewegliche Verbindung wird durch eine Kopplungsnute **48** am Steckmodul bzw. Adapter ermöglicht. Dabei bildet die Nute **48** mit dem Öffnungsrand der Öffnung **4** zumindest eine formschlüssige Verbindung. Die anderen Kopplungsteile **39** und **47** sorgen für eine bessere Einführung und Halterung des Steckmoduls **54** in der Vorrichtung. Eine Noppe **49** auf der Klappenoberseite sorgt für einen Abstand zur Steckadapteroberfläche. Dieser Abstand ist von Vorteil, wenn anstelle des Steckmoduls hier Instrumentenspitzen eingebracht sind um diese zu reinigen. Das Steckmodul **54** besitzt eine eingeschraubte Spüllanze **43** mit seitlichen Löchern **44** in die über den Zuführungskanal **25** Flüssigkeit austreten kann, um beispielsweise hierdurch aufgesteckte große Innenkanälen von Trokare oder andere medizinischen Hohlrauminstrumente zu reinigen. Die **Pfeile d** deuten nur die Fließrichtung von Medien an, die über den Rohrverteiler **9** zugeführt werden. Zum besseren Medieneintritt ist der eingeschraubte vordere Adapterteil **55** angeschrägt. Weitere **Pfeile a, e** und **f** zeigen die Beweglichkeit des Steckmoduls in der Vorrichtung.

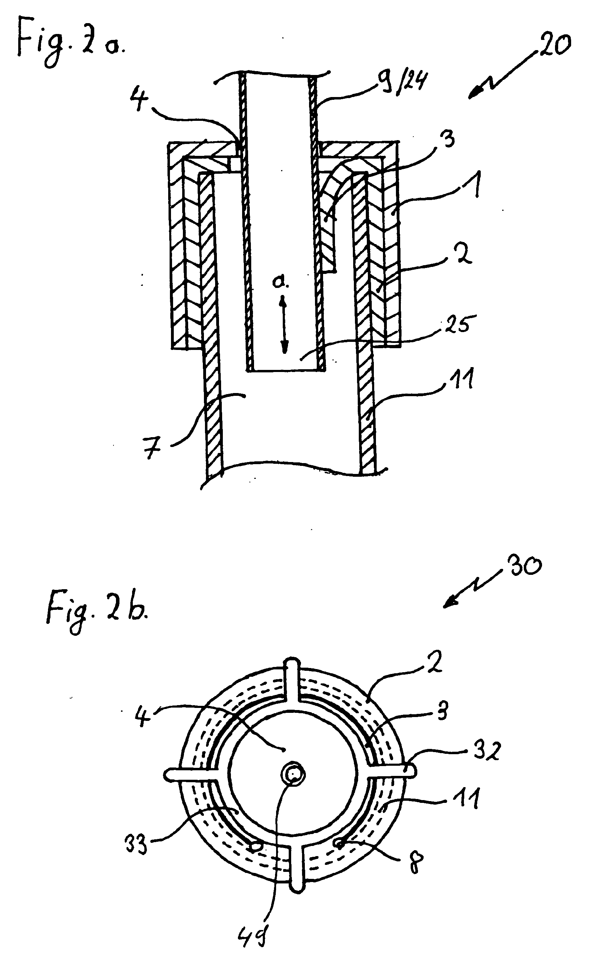

Figur 2a. zeigt eine Schnittdarstellung einer erfinderischen Spülvorrichtung im geöffneten Zustand in Verbindung mit einem Rohrende. Mit **20** ist eine Spülvorrichtung gezeigt. Dabei wird diese aus zwei Kappen **1** und **2** gebildet, welche über ein Adapter, Kopplungsteil oder ein Rohrende **11** gestülpt sind. Alternativ kann dies ein Adapterteil oder ein Schlauchanschlussteil sein. Dargestellt ist, wie durch Einbringen (vor und zurück bewegen, gezeigt durch den **Pfeil** a) eines Rohrverteilers **9** oder eines Produktes **24** bzw. medizinisches Instrumentes oder eines Kopplungsteils sich die Klappe **3** in der Kappe **2** nach innen öffnet. Ist die Klappe **3** in den Raum **7** der Vorrichtung hinein geöffnet, kann Flüssigkeit durch den Kanal **25** herausströmen. Die obere Lochkappe **1** dient dabei als Anschlagsbegrenzung für die Klappe 3 sowie auch als Dicht- und Halterungskappe für die über die Öffnung **4** einzusteckenden Produkte **24** oder Rohrverteiler **9.** Dabei dichtet der Randbereich der vorzugsweise elastischen und dehnbaren Öffnung **4** den Rohrverteilerschaft **9** zumindest durch einen Formschluss ab. Die Dichtung sollte so ausgelegt sein, dass z. B. die Produkte nicht allein durch den Flüssigkeitsdruck herausgedrückt werden können. Dabei kann die Öffnung **4** sternförmig oder anders ausgeführt sein um eine bessere Produktreinigung dort im Schaftbereich zu erzielen, wo eine Produktdichtung oder -halterung erreicht wird. Um zwischen Klappe 3 und Produkt 24 einen Abstand zu erzielen, kann auf der Klappe 3 eine Noppe vorhanden sein, damit diese beabstandet vom Produktschaft gehalten wird.

Figur 2b. zeigt eine Draufsicht eines Spülplatzes oder Spülanschlusses mit einer Spülvorrichtung in Verbindung mit einem Klappenanschlag. Mit **30** ist ein verschließbarer Spüldapter z. B. für Hohlrauminstrumente dargestellt. Dabei ist eine Kappe **2** mit einer Noppe **49** oder alternativ eine Scheibe mit einer integrierten Klappe **3** gezeigt, welche auf oder über einem Adapter oder ein Rohrende **11** gestülpt ist. Da diese Vorrichtung vorzugsweise zur Produktreinigung einsetzbar ist, verbessert die Noppe **49** als Abstandselement die Produktreinigung. Alternativ kann die Oberfläche der Klappe auch mehrere Noppen o. dgl. aufweisen. Alternativ kann die Kappe oder Scheibe auch durch ein Bürsten oder Fächerelement ersetzt sein. Die Kappe **2** hat Löcher **8,** um ein Ausreißen der Klappe **3** zu verhindern. Um die Kappe **2** zu halten und einen Anschlag für die untenliegende Klappe **3** zu ermöglichen wurde ein Drahtelement **32** mit einer inneren Anschlagbegrenzung in Form eines Rings **33** aufgesetzt. Das Anschlagselement **33** ist so gefertigt, dass dieses die Kappe **3** nach oben hin begrenzt. Durch den Anschlagsring **33** kann hier ein Instrument eingeführt werden. Die untere Klappe **3** wird sich dabei öffnen. Das Ringelement verhindert auch, dass sich die Klappe nach oben öffnen kann. Diese Spülvorrichtung lässt Flüssigkeit durch, begrenzt jedoch die Durchtrittsmenge erheblich.

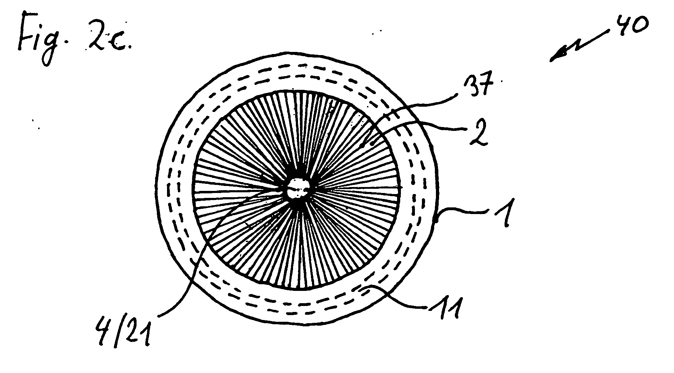

Figur 2c. zeigt eine Draufsicht eines Spülplatzes oder Spülanschlusses mit einem Büstenelement. Dabei stellt **40** zumindest einen Spülanschluss oder eine Spülhülse zum Spülen von medizinischen Instrumenten mit unterschiedlichen Querschnittsformen und -größen dar. Die Bürsten **37** oder alternativ hier vorhandene Fächerscheiben sind zu einem Bürstenelement **2** vereinigt. Dabei liegen viele Bürsten **37** übereinander und verringern so die Durchtrittsfläche für Flüssigkeiten. Das Bürstenelement **2** wird dabei über eine Lochkappe 1 über ein Rohrende **11** bzw. einen Adapter etc. fixiert. Mittig ermöglichen die Büsten eine Öffnung **4** bzw. **21.** Hier kann von oben ein Instrument eingebracht werden. Dabei biegen sich die Bürsten nach unten, streifen grobe Verunreinigungen ab und halten gleichzeitig das Instrument. Die Lochkappe **2** oder alternativ ein Deckel dient dabei als Anschlagsbegrenzung für die Bürsten **37** und verringert so, dass sich die Bürsten zu leicht nach außen biegen, wenn Flüssigkeit herausströmt. Die Ausgestaltung **40** zeigt, dass über Bürsten besonders Produkte mit unterschiedlichen Querschnitten eingebracht werden können.

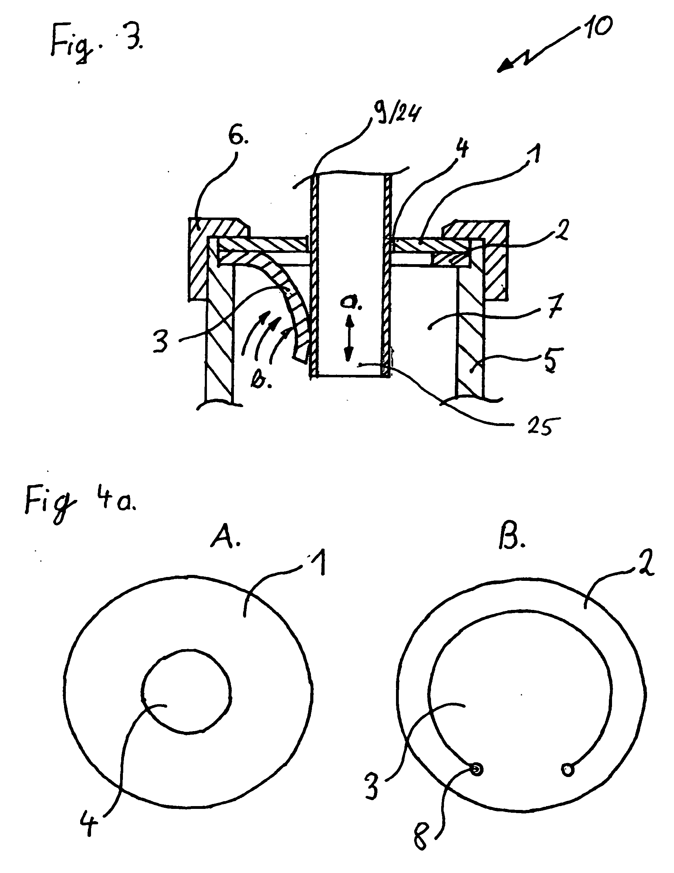

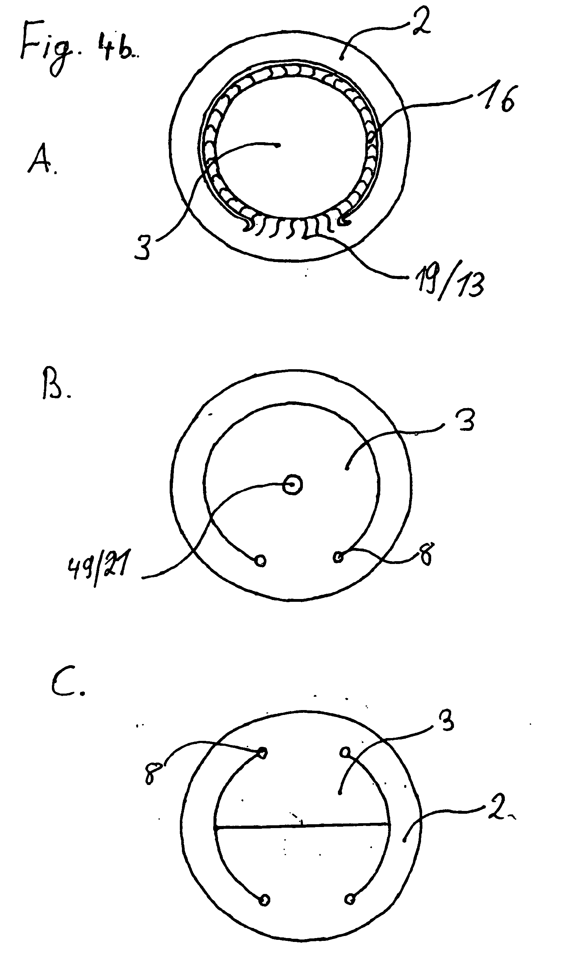

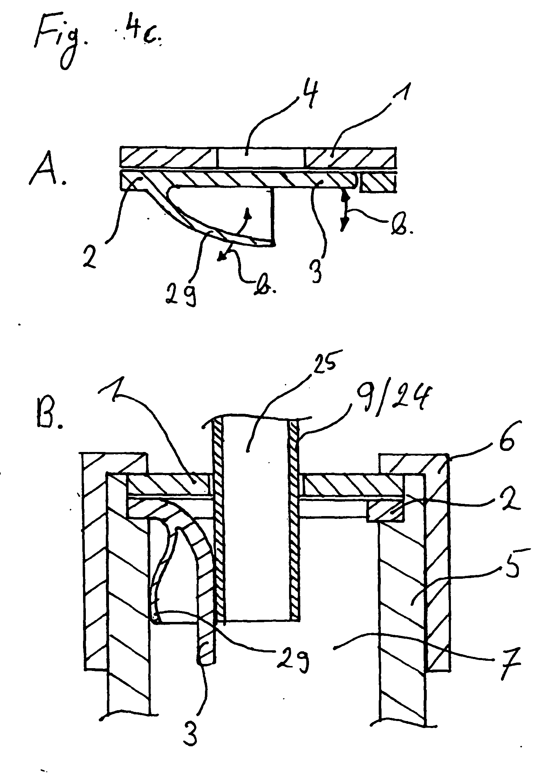

Figur 3. zeigt eine Schnittdarstellung einer erfinderischen Spülvorrichtung im geöffneten Zustand in Verbindung mit einem Zylinder. Die Vorrichtung **10** besteht aus mehreren Teilen. Einem unteren zylindrischen Körper **5** mit einem Außengewinde mit einem Schraubdeckel **6,** der eine große Öffnung aufweist. Der Schraubdeckel **6** presst eine untere Scheibe **2** mit einer beweglichen Klappe **3** und eine oben aufliegende Lochscheibe **1** mit einer Öffnung **4** aufeinander. Über die Öffnung **4** ist hier ein Rohrverteiler **9** oder ein Hohlrauminstrument **24** eingebracht. Dabei soll die obere Scheibe **1** eine geeignete Dichtung herstellen. Der **Pfeil a** deutet an, das z. B. der Rohrverteiler lösbar verbunden ist und in den Zylinder vor und zurück bewegt werden kann und dabei die Klappe **3** sich in den Raum **7** öffnet. Durch die Rückstellkraft der Klappe (dargestellt durch die **Pfeile b)** wird der eingebrachte Rohrverteiler oder werden die Produkte zusätzlich gehalten. Hier wäre es von Vorteil, wenn die untere Scheibe eine Doppelklappe (siehe Figur 4b, C) oder vier Klappen besitzen würde. Erst nachdem die Klappe **3** sich geöffnet hat, kann Flüssigkeit über den Kanal **25** herausströmen. Alternativ kann die oben aufliegende Scheibe auch mehrere Durchbrüche oder Löcher besitzen, um hier z. B. kleine Bohrer einzubringen und zu halten.

Figur 4a. zeigt eine Draufsicht einer Lochscheibe und einer Scheibe mit einer beweglichen Klappe. Skizze **A.** zeigt dabei eine Lochscheibe **1** mit einer runden Öffnung **4.** Skizze **B.** zeigt eine Scheibe 1, bei der eine Klappe **3** ausgestanzt wurde. Um ein Ausreißen zu vermeiden, befinden sich an den endständigen Schnittstellen Löcher **8.** Die Scheibendicken sind abhängig vom vorhandenen Flüssigkeitsdruck im System oder von der Art der benötigten Dichtigkeit.

Figur 4b. zeigt Draufsichten unterschiedlicher Klappendesigns. In **A** ist eine Scheibe **2** mit einer Klappe **3** gezeigt, welche einen umlaufenden Rand **16** aufweist der vorzugsweise nach unten angeordnet ist. Dadurch entsteht ein tellerförmiger Rand, um den Verschluss und die Klappenstabilität der Klappe **3** zu verbessern sowie einen besseren Verschluss über den Flüssigkeitsdruck zu ermöglichen. Damit die Klappe fest mit der Scheibe **2** verbunden ist, besitzt diese eine weitere Materialverstärkung **13** bzw. Materialerhöhung **19.** Alternativ zur Umrandung 16 kann die Klappenoberfläche ein oder beidseitig noppenartige Erhöhungen besitzen oder eine Kombination aus beidem aufweisen. In B. ist wiederum eine Scheibe 2 gezeigt. Diese besitzt jedoch ein mittiges Loch 21 oder zumindest eine zentral angeordnete Noppe 49, um die Belastung auf die Klappe zu reduzieren oder einen Abstandshalter zu besitzen. Als Ausreißschutz sind am Ende des Schnittes Löcher 8 eingebracht. Die Darstellung C. zeigt ein zweiflügeliges Klappendesign. Die beiden Klappen 3 sind mit der Scheibe 2 verbunden. Als Ausreißschutz sind vier Löcher **8** eingebracht oder können alternativ Materialverdickungen vorhanden sein.

Figur 4c. zeigt eine Schnittdarstellung einer Klappe mit einer taschenartigen Erhebung ohne und mit einem Rohrende oder Zylinder. In **A** ist eine Klappenvorrichtung aus zwei Scheiben gezeigt. Die unten liegende Scheibe **2** besitzt eine bewegliche Klappe **3,** welche auf der unteren Seite eine zusätzliche Tasche **29** hat. Oben aufliegend befindet sich eine Lochscheibe **1** mit einer Öffnung **4.** Die **Pfeile b** sollen verdeutlichen, dass sowohl die Klappe **3** wie auch die Tasche **29** in der Ausformung änderbar und beweglich sind. Die Abbildung **B.** gibt schematisch wieder, wie sich die Tasche verhält, wenn ein Produkt **24** oder ein Rohrverteiler **9** in die Vorrichtung eingebracht wurde. Damit ein Klappenverschluss zustande kommt, besitzt die Vorrichtung einen äußeren Deckel **6,** der beispielsweise mit einem Zylinder oder Rohr **5** lösbar verbunden ist. Dieser presst beide Scheiben **1** und **2** zusammen und ermöglicht ein einfaches Austauschen dieser. Die Klappe **3** kann sich dabei in den Raum **7** bewegen. Der Anpressdruck der Klappe an dem Rohrverteiler **9** oder an einem Produkt **24** wird von der Tasche **29** unterstützt und verhindert, dass sich die Klappe **3** ganz an die Zylinder- oder Rohrwandung anlegt. Liegt die Tasche **29** nicht zu eng an, wird sichergestellt, dass sich die Klappe **3** sicher wieder schließt, wenn z. B. das Produkt **9** wieder herausgezogen wird.

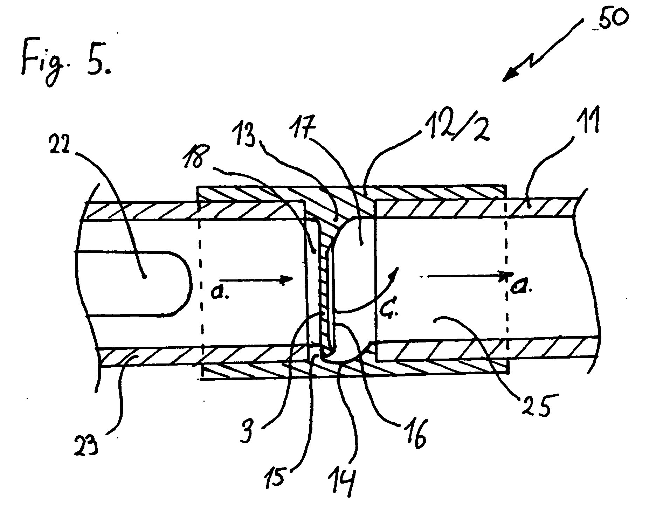

Figur 5. zeigt eine Schnittdarstellung eines Rohr- oder Schlauchverbinders mit integrierter Klappe. Mit **30** ist ein Rohr- oder Schlauchverbinder gezeigt, der eine integrierte Klappe **3** mit einer Randerhöhung **16** besitzt. Diese Vorrichtung **30** wird zum Verbinden von Rohrenden genutzt und kann auch zur Produktspülung eingesetzt werden. Der Rohr- oder Schlauchverbinder **12** enthält eine Klappe **3,** die sich nur zu einer Seite hin öffnen kann. Um dies zu ermöglichen, wurde durch eine Querschnittserweiterung ein Vorraum **17** geschaffen. Desweiteren wurde eine Vertiefungsmulde **14** und ein Anschlag **15** für die Klappenbeweglichkeit eingebaut. Der Verbinder **12** bzw. **2** (auch als Verbindungskappe zu verstehen) besitzt endständig röhrförmige Ansatzteile, um hier Instrumentenhalterungen **23** mit großen Durchbrüchen **22,** Schläuche, Rohrenden **11** oder beispielsweise auch Gefäßimplantate miteinender zu verbinden. In der Abbildung **30** wurde ein Rohrende **11** mit einer Instrumentenhalterung **23** verbunden. Damit die Anschlüsse die Beweglichkeit der Klappe 3 nicht behindern, sind Räume vor **18** und **17** nach der Klappe **3** vorgesehen. Die **Pfeile a** geben an, das hier ein Hohlrauminstrument eingebracht werden kann. Die Beweglichkeit der Klappe **3** ist durch **Pfeil c** dargestellt. Damit die Klappenstabilität gewährleistet ist, wurde eine Materialverstärkung **13** eingebracht. Fließt hier Flüssigkeit über den Kanal **25** in Pfeilrichtung **a,** wird sich die Klappe **3** in den Raum **17** und **25** öffnen und wieder verschließen, wenn die Flüssigkeit zurückfließt.

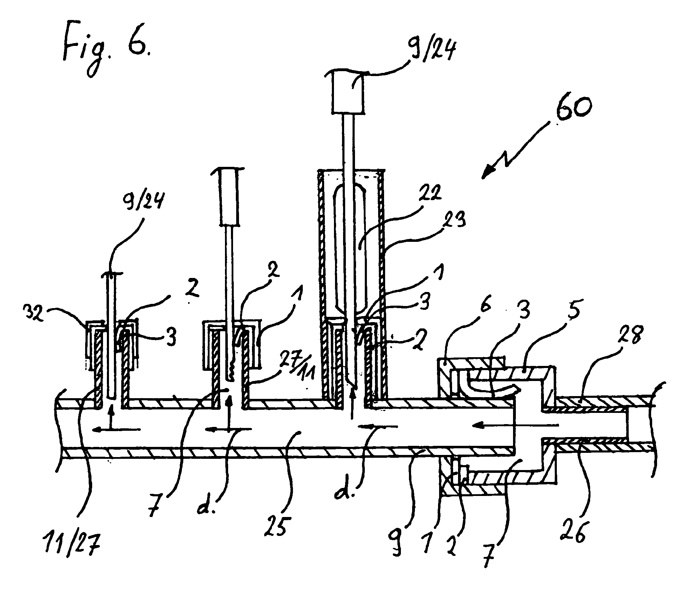

Figur 6. zeigt eine Schnittdarstellung einer modular aufgebauten Spülvorrichtung mit unterschiedlichen Klappenverschluss- und Produkthaltevorrichtungen. Die Abbildung **40** zeigt eine modulare Spülvorrichtung, welche aus mehreren verschließbaren Komponenten aufgebaut ist. Eine Komponente stellt einen Spülzylinder bzw. Steckzylinder **5** dar, der über einen Deckel **6** zwei Scheiben **1** und **2** fixiert. Eine Scheibe besitzt eine verschließbare Klappe **3.** Damit über den Steckzylinder **5** Flüssigkeit zugeführt werden kann, besitzt dieser einen Schlauchansatz **26.** An diesen kann ein Schlauch **28** angeschlossen werden und diese Zuleitung wiederum mit einem Hauptmedienverteiler verbunden sein. In den Raum **7** des Steckzylinders **5** wurde eine Rohrverteilerleiste **9** mit Ansätzen **27** eingebracht. Dieser Vorrichtungsteil verschließt sich selbständig, wenn der Rohrverteiler **9** wieder herausgezogen wird. Damit z. B. medizinische Instrumente **24** gereinigt werden können, befinden sich auf dem Rohrverteiler **9** zylindrische Ansätze 27 oder Rohrteile **11.** Ein Ansatz besitzt dabei nur eine Spülkappe **2** und diese hat eine Klappe **3** integriert, welche sich durch ein Instrument **24** öffnet. Dabei kann diese als Rohrverbinder **11,** aber auch als eine verschraubte Kappe **2** ausgeführt sein oder von außen durch eine Klappenanschlagsbegrenzung (siehe Figur 2b) gehalten werden. Zwei weitere andere Ansätze **27** besitzen Kappen **1** und **2,** wobei die untere Kappe **2** eine bewegliche Klappe **3** aufweist. Hierüber können Produkte **24** eingesteckt werden. Dabei hält die äußere Kappe **1** das Produkt **24** fest und bildet einen dichtenden Verschluss. Um insbesondere das senkrechte Halten von längeren Produkten **24** zu ermöglichen, wurde zusätzlich an einem Ansatz **27** eine Verlängerungshülse **23** mit großen Durchbrüchen **22** über die Kappen **1** und **2** gesteckt. Die Durchbrüche **22** sollen dabei die Außenreinigung der Produktschäfte verbessern. Die Dichtkappen **1** müssen dabei so dichten, dass Flüssigkeit (durch **Pfeile d** dargestellt) in ausreichender Menge, welche durch den Kanal **25** in die Räume **7** strömt, in die inneren Instrumentenkanäle gelangt. Die hier gezeigt Spülvorrichtung kann aber auch anders aufgebaut sein. Eine weitere mögliche Ausgestaltung ergibt sich, wenn ein Halterungsblock eingesetzt wird, in dem links und rechts ein Rohrverteiler eingebracht werden kann. Dabei kann vorgesehen sein, die Rohrverteiler durch Schrauben mit dem Block lösbar zu verbinden. Der Block wiederum könnte Steckanschlüsse oder weitere Verschraubungen besitzen, um eine Verbindung mit einem Hauptmedienverteiler zu ermöglichen. Die gezeigte Vorrichtung oder ähnliche Ausgestaltungen können einfach in einem Siebkorb eingelegt oder mit diesem lösbar verbunden werden. Damit ist sichergestellt, dass hier vorhandene Hohlraumprodukte durchspült werden.

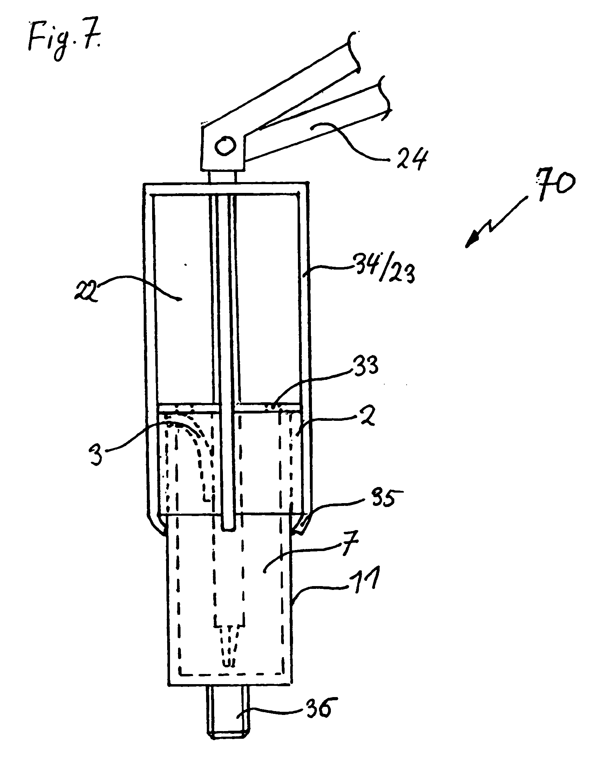

Figur 7. zeigt die Seitenansicht eines Spülplatzes für medizinische Instrumente mit einer zusätzlichen Instrumentenhalterung. In **70** ist ein Spülplatz für medizinische Instrumente gezeigt. Das Instrument **24** und die Kappe **2** wird dabei zusätzlich über eine Halterungsvorrichtung **34** bzw. **23** mit großen Durchbrüchen gehalten. Zusätzliche Biegungen **35** an der Halterungsvorrichtung sorgen für einen optimalen Halt der Vorrichtung. Damit das Instrument **24** an der Spitze oder des inneren Instrumentenkanals gereinigt werden kann, wurde eine Kappe **2** mit einer Klappe **3** über eine Hülse **11** gestülpt. Diese Hülse **11** weist nach unten ein Gewinde **36** oder einen Steckanschluss auf, damit diese mit einem Rohrverteiler oder einem Schlauch verbunden werden kann. Dabei öffnet das eingebrachte Instrument **24** die **Klappe 3** in den Raum **7** der Hülse **11.** Damit sich die Klappe **3** nach der Entnahme des Instrumentes nicht nach außen öffnet, liegt ein Anschlagsring **33** auf der Kappe **2** auf.

## Patentansprüche

1. Verschließbare Vorrichtung zum Spülen, Anschließen und Koppeln, die insbesondere im Bereich der medizinischen und industriellen Produktaufbereitung zum Durchleiten von Flüssigkeiten, zum Einsatz kommt und dabei wenigstens eine Öffnung (4) zum Durchtritt von Medien sowie ein Anschlag-, Dicht- und/oder Halteelement (1, 6, 15, 33) aufweist, **dadurch gekennzeichnet, dass** der Durchtritt von Medien über die Öffnung (4) durch zumindest ein elastisches Klappen- (3, 29), Bürsten- (37) oder Fächerelement reduziert oder verhindert ist, um durch Einbringen eines Produktes (24), Rohrverteilers (9) oder eines Adapters (11, 54) ein oder mehrere elastische Elemente nach unten in einen Kanal (7) oder Raum zu biegen und **dadurch** zumindest eine bewegbare Verbindung zur Flüssigkeitsweiterleitung mit einem Kopplungsteil (38, 47, 48, 54) oder einem Produktteil (24) und/oder eine Produktreinigung in oder über die Vorrichtung herzustellen.

2. Verschließbare Vorrichtung nach Anspruch 1, **dadurch gekennzeichnet, dass** das Anschlag-, Dicht- und/oder Halteelement (1, 6,15, 33) zumindest mit einer Lochscheibe, einer Kappe (1), einem Absatz (15) oder einem Drahtelement (33) gebildet wird und durch einen elastischen Öffnungsbereich (4) unterschiedliche Produktgeometrien und Produktquerschnitte in die Vorrichtung eingebracht werden können und sich die beweglichen Teile der Vorrichtung (3, 29, 37) wieder nach der Entnahme zumindest durch den Flüssigkeitsstrom zurückbewegen.

3. Verschließbare Vorrichtung nach Anspruch 1 und 2, **dadurch gekennzeichnet, dass** zumindest durch den Einsatz von elastischen Halte- und Dichtelementen (1), die eingebrachten Produkte (9, 24, 54) in der Vorrichtung zumindest drehbar sind und je nach Ausführung auch seitlich in mehreren Richtungen sowie vor- und zurückbewegbar sind.

4. Verschließbare Vorrichtung nach Anspruch 1 bis 3, **dadurch gekennzeichnet, dass** die Vorrichtung modular aufgebaut ist und zumindest die Scheiben, Kappen (1, 2), Bürstenelemente (37) oder Halteelemente (23, 32, 34) oder Haltefedern (51) austauschbar sind.

5. Klappen nach einem der Ansprüche 1 bis 4, **dadurch gekennzeichnet, dass** die Klappen (3) einen erhöhten Rand (16), Noppen (49), Taschen (29) einen und/oder eine Materialverstärkung (13, 19) und/oder ein Loch (21) besitzen.

6. Verschließbare Vorrichtung nach einem der Ansprüche 1 bis 5, **dadurch gekennzeichnet, dass** zumindest eine Scheibe (1) und eine Scheibe (2) mit einer Klappe (3, 29) in einem Zylinder (5) oder Block über einen Deckel (6) fixiert ist oder ein Adapter (11) oder Rohrende (27) eine Kappe (2) mit einer Klappe (3) aufweist und diese mit einer überstülpten Kappe (2) fixiert ist.

7. Verschließbare Vorrichtung nach einem der Ansprüche 1 bis 6, **dadurch gekennzeichnet, dass** eine Produktreinigung in der Vorrichtung erfolgt und/oder zusätzlich über Bürsten (37) oder Fächerscheiben die Produktaußenflächen (9, 24), zumindest beim aus- und einführen mechanisch gereinigt werden.

8. Verschließbare Vorrichtung nach einem der Ansprüche 1 bis 7, **dadurch gekennzeichnet, dass** mit zumindest einem Dicht- und Halteelement (1) und einer Öffnung (4) in Verbindung mit einem Steckmodul (54) eine kraft- und/oder formschlüssige Kopplung mit einer Halterungsnute (48) gebildet wird, um über angeschlossene Luer Lock Adapterteile (42, 45), Spüllanzen (43), Schlauchverbinder (41) oder dergleichen die Anschlussmöglichkeiten und Nutzung der Vorrichtung zu erweitern.

9. Verschließbare Vorrichtung nach einem der Ansprüche 1 bis 8, **dadurch gekennzeichnet, dass** eine Vorrichtung (20, 30, 40, 50, 60, 70, 80) oder eine Kombination aus diesen eingesetzt wird und diese mit Rohrverteilern (9) oder Spülleisten, Spülzylindern (5), Maschinenleisten und/oder in Siebkörben oder auf Gestellen zumindest lösbar verbunden ist und/oder durch diese weitere Anschlussmöglichkeiten zumindest für medizinische Instrumente, Schläuche, Halterungsblöcke o. dgl. entstehen.

10. Verschließbare Vorrichtung nach einem der obigen Ansprüche 1 bis 9, **dadurch gekennzeichnet, dass** in Verbindung mit einem Dreharm, Zylinder (5), Adapter (11), Verbinder (12) oder einem Spülrohranschluss (27) eine Kappe (1) eine untere Scheibe mit zumindest einer Klappe (3) oder ein Bürstenelement (2, 37) oder ein Fächerscheibenelement oder eine Kombination aus diesen nach oben begrenzt und zusätzlich ein Filterelement enthält.

11. Spülverfahren in Verbindung mit Flüssigkeits- oder Lösungskreisläufe, dass insbesondere in Reinigungsmaschen in der Medizin-, Labor- und Industrietechnik Anwendung findet und **dadurch gekennzeichnet ist, dass** in einem Flüssigkeitskreislauf zumindest ein Spülplatz oder ein Spülanschluss zumindest eine verschließbare Vorrichtung (10, 20, 30, 40, 50, 60, 70, 80) besitzt, die sich durch mindestens eine Klappe (3) oder über Bürsten (37) oder Fächerscheiben durch den Flüssigkeitsstrom oder von alleine schließt und eine Produktspülung erfolgt, wenn dieser Spülplatz oder Spülanschluss zumindest durch einen eingebrachten Kanal (25) oder ein Produkt (9, 24, 54) belegt ist und eine reduzierte Flüssigkeitsmenge oder keine Flüssigkeit austritt, wenn dieser nicht belegt ist.

12. Spülverfahren nach Anspruch 11, **dadurch gekennzeichnet ist, dass** durch die Entnahme eines Rohrverteilers (9), eines Adapters (11, 54), eines zu reinigendes Produkt (24) oder dergleichen aus einem Spül- oder Kopplungsplatz der Reinigungsdruck in einem Flüssigkeitskreislauf nicht signifikant abfällt und damit der Reinigungserfolg zumindest in einer Reinigungsmaschine und/oder in einem Ultraschalbad erhalten bleibt.

13. Spülverfahren nach Anspruch 11 und 12, **dadurch gekennzeichnet, dass** zumindest Hohlraumprodukte (24) in einem Siebkorb oder auf einem Gestell verbleiben und hier die Produktkanäle oder Produktspitzen über vorhandene verschließbare Spülplätze gereinigt werden und kein Umpacken und Umsortieren auf andere Maschinenwagen o. dgl. erfolgt.

14. Spülverfahren nach einem der Ansprüche 11 bis 13, **dadurch gekennzeichnet, dass** Halterungsblöcke, Rohrverteiler (9), Zylinder (5), Adapter (11, 27, 54) oder zusätzliche Produkthalterung (34, 23) vorhanden sind und diese fest oder mobil in Siebkörbe oder auf Gestelle und/oder in Ultraschallbädern, Maschinen oder einen Maschinenwagen installiert oder hier nur eingelegt sind.

15. Spülverfahren nach einem der Ansprüche 11 bis 14, **dadurch gekennzeichnet, dass** durch die Anzahl verschließbarer Spülvorrichtungen an Rohrverteilern in einer Maschine der Flüssigkeitskreislauf und der Flüssigkeitsdruck änderbar und beeinflussbar wird und **dadurch** zusätzliche Anschluss- und Spülmöglichkeiten für Instrumente (24) und Kopplungsteile (11, 54) entstehen.

## Claims

1. Closable device for rinsing, connecting and coupling the passage of liquids especially in the area of medical and industrial product preparation, to be used with and have at least one opening (4) for the passage of media as well as an end, sealing and/or retaining element (1, 6, 15, 33), **characterised in that** the passage of media through the opening (4) is reduced or impeded by at least one elastic flap (3, 29), brush (37) or fan element, allowing by the introduction of a product (24), pipe distributor (9) or an adapter (11, 54) one or more elastic elements to bend downwards into a channel (7) or space and in so doing make at least one moveable connection for liquid transfer with a coupling part (38, 47, 48, 54) or a product part (24) and/or a product cleaning in or through the device.

2. Closable device according to claim 1, **characterised in that** the stop, sealing and/or retaining element (1, 6,15, 33) will be made up of at least a perforated disc, a cap (1), a section (15) or a wire element (33) and through an elastic opening range (4) different product geometries and product cross-sections can be brought into the device and the movable parts of the device (3, 29, 37) will move back after withdrawal due at least to the flow of liquid.

3. Closable device according to claims 1 and 2, **characterised in that** at least by the use of elastic retaining and sealing elements (1) the introduced products (9, 24, 54) in the device are at least rotatable and depending on design can also move laterally in a number of directions as well as forward and backwards.

4. Closable device according to claims 1 to 3, **characterised in that** the device is built in a modular manner and that at least the discs, caps (1, 2), brush elements (37) or stop elements (23, 32, 34) or retaining springs (51) are replaceable.

5. Flaps according to one of the claims 1 to 4, **characterised in that** the flaps (3) possess a raised edge (16), studs (49), pockets (29) one and/or a material strengthening (13, 19) and/or a hole (21).

6. Closable device according to one of the claims 1 to 5, **characterised in that** at least one disc (1) and a disc (2) with a flap (3, 29) is fixed in a cylinder (5) or block via a cover (6) or an adapter (11) or pipe end (27) has a cap (2) with a flap (3) and this is fixed with a pull-on cap (2).

7. Closable device according to one of the claims 1 to 6, **characterised in that** a product cleaning is carried out in the device and/or additionally the external product surfaces (9, 24) are mechanically cleaned by brushes (37) or fan blades at least during insertion and removal.

8. Closable device according to one of the claims 1 to 7, **characterised in that** with at least one sealing and one retaining element (1) and an opening (4) in connection with a plug-in module (54) a force and/or a form-fitting coupling will be made with a mounting slot (48) to expand the connection options and the use of the device by connected Luer Lock adapter parts (42, 45), rinsing lances (43), hose connectors (41) or the like.

9. Closable device according to one of the claims 1 to 8, **characterised in that** a device (20, 30, 40, 50, 60, 70, 80) or a combination of these will be implemented and connected with pipe distributors (9) or rinsing lines, rinsing cylinders (5), machine lines and/or at least loosely connected in screen baskets or racks and/or through these arise further connection options at least for medical instruments, hoses, retaining blocks or the like.

10. Closable device according to one of the above claims 1 to 9, **characterised in that** in conjunction with a rotating arm, cylinder (5), adapter (11), connector (12) or a rinsing tube connector (27) a cap (1) contains a lower disc with at least one flap (3) or a brush element (2, 37) or a fan blade element or a combination of the maximum number of these and additionally a filter element.

11. Rinsing method in connection with liquid or solution loops, that finds application in particular in cleaning meshes in medical, laboratory and industrial technology and is **characterised in that** in a liquid loop at least one rinsing area or a rinsing connection has at least one closable device (10, 20, 30, 40, 50, 60, 70, 80) that closes due to the liquid flow or closes itself with at least one flap (3) or via brushes (37) or fan blades and a product rinsing follows, when this rinsing place or rinsing connection is occupied at least by an introduced channel (25) or a product (9, 24, 54) and a reduced quantity of liquid or no liquid escapes when this is not occupied.

12. Rinsing method according to claim 11, **characterised in that** through the withdrawal of a pipe distributor (9), an adapter (11, 54) or a product which is to be cleaned (24) or such like from a rinsing or coupling place the cleaning pressure in a liquid loop does not drop significantly and therefore the cleaning success at least in a cleaning machine and/or in a ultrasonic bath remains the same.

13. Rinsing method according to claims 11 and 12, **characterised in that** at least cavity products (24) remain in a screen basket or on a frame and here the product channels or product lines are cleaned by available closable rinsing spaces and no unpacking and re-sorting takes place on other machine carriages or the like.

14. Rinsing method according to one of the claims 11 to 13, **characterised in that** the retaining blocks, pipe distributor (9), cylinder (5), adapter (11, 27, 54) or additional product mounts (34, 23) are available and these are installed as fixed or movable in screen baskets or on frames and/or in ultrasonic baths, machines or a machine carriage or only laid here.

15. Rinsing method according to one of the claims 11 to 14, **characterised in that** the number of closable rinsing devices on pipe distributors in a machine of the liquid circuit and the liquid pressure will be changed and influenced and thus additional connection and rinsing options for instruments (24) and coupling parts (11, 54) arise.

## Revendications

1. Dispositif verrouillable de rinçage, raccordement et couplage, principalement utilisé dans la préparation de produits médicaux et industriels qui ont pour but l'entraînement de liquides. Il présente par conséquent au moins une ouverture (4) pour le passage de fluides ainsi qu'un organe de butée, d'étanchéité et/ou d'arrêt (1, 6, 15, 33). Il est **caractérisé par le fait que** le passage des fluides par l'ouverture (4) est réduit ou empêché par au moins un organe élastique à clapet (3, 29), à brosses (37) ou à denture de manière à ce que, après introduction d'un produit (24), d'une tubulure (9) ou d'un adaptateur (11, 54), un ou plusieurs éléments élastiques se replient dans un canal (7) ou un compartiment pour ainsi créer une connexion mobile entre la conduite du fluide et un organe de couplage (38, 47, 48, 54), une pièce du produit (24), et/ou un dispositif de nettoyage dans ou à l'extérieur du dispositif.

2. Le dispositif verrouillable tel que décrit à la revendication 1 est **caractérisé par le fait que** l'organe de butée, d'étanchéité et/ou d'arrêt (1, 6, 15, 33) doit être au moins constitué d'un disque à trous, d'un cache (1), d'un butoir (15) ou d'un élément fileté (33), que différentes géométries et sections de produits peuvent être introduites dans le dispositif au travers d'une zone d'ouverture élastique (4), et que les pièces mobiles du dispositif (3, 29, 37) peuvent au moins être ramenés par le flux liquide une fois le prélèvement terminé.

3. Le dispositif verrouillables tel que décrit aux revendications 1 et 2 est **caractérisé par le fait que** les produits introduits (9, 24, 54) dans ledit dispositif sont au moins pivotants grâce à la mise en oeuvre d'organes d'arrêt et d'étanchéité élastiques (1) et, selon le modèle, qu'ils sont mobiles vers l'avant, vers l'arrière et latéralement dans plusieurs directions.

4. Le dispositif verrouillable tel que décrit aux revendications 1 à 3 est **caractérisé par le fait que** le dispositif est conçu de façon modulaire et que les disques, les caches (1, 2), les organes à brosses (37), les organes d'arrêt (23, 32, 34) ou les ressorts de retenue (51) doivent pouvoir être remplacés.

5. Les clapets tels que décrits aux revendications 1 à 4 sont **caractérisés par le fait que** lesdits clapets (3) possèdent un bord surélevé (16), des aspérités (49), des poches (29) et/ou un matériau de renfort (13, 19) et/ou un trou (21).

6. Le dispositif verrouillable tel que décrit aux revendications 1 à 5 est **caractérisé par le fait qu'**au moins un disque (1) et un cache (2) à clapet (3,29) sont fixés sur un couvercle (6) dans un cylindre (5) ou un bloc, ou qu'un adaptateur (11) ou un manchon (27) présente un cache (2) à clapet, lequel est fixé à un cache rabattable (2).

7. Le dispositif verrouillable tel que décrit aux revendications 1 à 6 est **caractérisé par le fait que** le nettoyage d'un produit est effectué dans ledit dispositif et/ou que les surfaces extérieures du produit (9, 24) sont au moins nettoyées mécaniquement par des brosses (37) ou des disques à denture lors de l'extraction ou de l'introduction du produit.

8. Le dispositif verrouillable tel que décrit aux revendications 1 à 7 est **caractérisé par le fait qu'**au moins un organe d'étanchéité ou d'arrêt (1) ainsi qu'une ouverture (4) connectée à un module enfichable (54) constituent un couplage mécanique ou par adhérence avec encoche de fixation, de manière à améliorer les possibilités de raccordement ainsi que l'utilisation du dispositif au moyen d'adaptateurs Luer Lock (42, 45), de lances de projection (43), de raccords de tuyauterie souple (41) ou de dispositifs similaires.

9. Le dispositif verrouillable tel que décrit aux revendications à 1 à 8 est **caractérisé par le fait qu'**un dispositif (20, 30, 40, 50, 60, 70, 80) ou une combinaison de ces dispositifs est mis en oeuvre et relié à des tubulures (9) ou des barres/cylindres de rinçage (5), des barres de machines, dans des paniers ou sur des supports, et/ou que ceux-ci permettent d'obtenir d'autres possibilités de raccordement au moins pour des instruments médicaux, des flexibles, des blocs de montage...

10. Le dispositif verrouillable tel que décrit aux revendications 1 à 9 est **caractérisé par le fait que**, en association avec un bras de levier, un cylindre (5), un adaptateur (11), un connecteur (12) ou un raccord de lance (27), un cache limite par le haut un disque inférieur avec au moins un clapet (3) ou un organe à brosses (2, 37) ou un organe à denture ou une combinaison de ceux-ci, et qu'il comporte en outre un élément filtrant.

11. Le procédé de rinçage qui, associé à des circuits de fluide ou de solution, entre principalement en oeuvre dans des dispositifs de nettoyage médicaux, de laboratoire et industriels, est **caractérisé par le fait que**, dans un circuit de fluide, au moins un espace ou raccord de rinçage doit au minimum disposer d'un dispositif verrouillable (10, 20, 30, 40, 50, 60, 70, 80) qui se ferme au minimum par le biais d'un clapet (3) ou de brosses (37) ou de rondelles à denture par le simple courant de fluide ou par lui-même et qui effectue un rinçage du produit lorsque cet espace ou raccord de rinçage est occupé par au moins un canal (25) ou un produit (9, 24, 54), et provoque l'écoulement d'une quantité réduite ou nulle de liquide lorsqu'il n'est pas occupé.

12. Le procédé de rinçage tel que décrit à la revendication 11 est **caractérisé par le fait que** retirer une tubulure (9), un adaptateur (11, 54), un produit à nettoyer (24) ou un dispositif similaire depuis un espace de rinçage ou de couplage ne provoque pas une diminution significative de la pression de nettoyage dans un circuit de fluide et, par conséquent, préserve le résultat du nettoyage dans au moins un nettoyeur et/ou un bain d'ultrason.

13. Le procédé de rinçage tel que décrit aux revendications 11 et 12 est **caractérisé par le fait qu'**au moins des produits sous vide (24) résident dans un panier ou sur un support, et que les canaux ou pointes de produit y soient nettoyés par le biais d'espaces de rinçage verrouillables disponibles et qu'aucun reconditionnement ou aucune transformation ne se produise dans d'autres cabines de commande mobiles ou dispositifs similaires.

14. Le procédé de rinçage tel que décrit aux revendications 11 à 13 est **caractérisé par le fait que** des blocs de montage, des tubulures (9), des cylindres (5), des adaptateurs (11, 27, 54) ou d'autres supports de produit (34, 23) sont prévus et sont soit installés soit simplement encastrés de manière fixe ou mobile dans des paniers ou sur des supports et/ou dans des bains d'ultrason, des machines ou dans une cabine de commande mobile.

15. Le procédé de rinçage tel que décrit aux revendications 11 à 14 est **caractérisé par le fait que** le circuit de fluide et la pression du fluide sont modifiables et influençables par le nombre de dispositifs de rinçage verrouillables installés sur la tubulure d'une machine, et que cela entraîne par conséquent des possibilités de raccordement ou de rinçage supplémentaires pour des instruments (24) et pièces de couplage (11, 54).
